# EUROPEAN PATENT SPECIFICATION

(11) **EP 1 155 696 B1**
(45) Date of publication and mention of the grant of the patent: **03.03.2004**
(21) Application number: 01202986.4
(22) Date of filing: 09.04.1998
(51) Int. Cl.: A61K 31/551, A61P 25/00

(54) **Use of olanzapine for the manufacture of a medicament for neuroprotection**
Verwendung von Olanzapin zur Herstellung eines Arzneimittels zur Neuroprotektion
Utilisation de l'olanzapine pour la fabrication d'un médicament pour la neuroprotection

(30) Priority: 15.04.1997 US 43094
(43) Date of publication of application: 21.11.2001
(62) Divisional of application: 98302789.7
(73) Proprietor: ELI LILLY AND COMPANY, Indianapolis, Indiana 46285 (US)
(72) Inventor: Bymaster, Franklin Porter, Brownsburg, Indiana 46112 (US); Tollefson, Gary Dennis, Indianapolis, Indiana 46236 (US)
(74) Representative: Pritchard, Judith

(56) References cited:
- EP-A- 0 733 635
- EP-A- 0 872 239
- EP-A- 1 181 935

## Description

This invention provides the use of 2-methyl-4-(4-methyl-1-piperazinyl)-10H-thieno[2,3-b][1,5] benzodiazepine, (hereinafter referred as "olanzapine") as a neuro-protective agent.

Neurodegenerative processes can involve diverse areas of the Central Nervous System (CNS). Neurodegeneration appears clinically as a breakdown of functionally connected neuronal circuits with corresponding alterations in the neurotransmitter system and morphological organization of the affected cell system.

The normal functioning of the CNS presupposes a well-balanced interaction between different biochemical and structurally linked neuronal systems. When one member of a neuronal circuit is altered in its structural or biochemical entity, an imbalance in the functional system results and a compensatory mechanism must be activated in order to maintain physiological equilibrium.

Certain degenerative diseases of the central nervous system are believed to be exacerbated or initiated by processes that result in the generation of reactive oxygen intermediates.

At present, the pharmacological therapy of neurodegenerative disorders is limited to symptomatic treatments that do not alter the course of the underlying disease. Meanwhile, because of the current dissatisfaction with the currently marketed treatments for the above-described indications within the affected population, the need continues for safer, better-calibrated drugs which will either slow the progress of or even prevent such neurodegeneration altogether.

This invention provides the use of olanzapine or a pharmaceutically acceptable salt or solvate thereof for the manufacture of a medicament for treating neurodegeneration in a mammal.

Olanzapine is of the formula 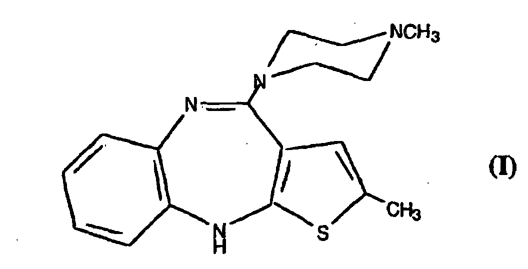 or an acid addition salt thereof.

It is especially preferred that olanzapine will be the Form II olanzapine polymorph having a typical x-ray powder diffraction pattern as represented by the following interplanar spacings:

| **d** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

A typical example of an x-ray diffraction pattern for Form II is as follows wherein d represents the interplanar spacing and I/I₁ represents the typical relative intensities:

| **d** | **I/I** _{**1**} |
|---|---|
| 10.2689 | 100.00 |
| 8.577 | 7.96 |
| 7.4721 | 1.41 |
| 7.125 | 6.50 |
| 6.1459 | 3.12 |
| 6.071 | 5.12 |
| 5.4849 | 0.52 |
| 5.2181 | 6.86 |
| 5.1251 | 2.47 |
| 4.9874 | 7.41 |
| 4.7665 | 4.03 |
| 4.7158 | 6.80 |
| 4.4787 | 14.72 |
| 4.3307 | 1.48 |
| 4.2294 | 23.19 |
| 4.141 | 11.28 |
| 3.9873 | 9.01 |
| 3.7206 | 14.04 |
| 3.5645 | 2.27 |
| 3.5366 | 4.85 |
| 3.3828 | 3.47 |
| 3.2516 | 1.25 |
| 3.134 | 0.81 |
| 3.0848 | 0.45 |
| 3.0638 | 1.34 |
| 3.0111 | 3.51 |
| 2.8739 | 0.79 |
| 2.8102 | 1.47 |
| 2.7217 | 0.20 |
| 2.6432 | 1.26 |
| 2.6007 | 0.77 |

The x-ray diffraction patterns set out herein were obtained using a Siemens D5000 x-ray powder diffractometer having a copper Kₐ radiation source of wavelength, 1 =1·541Å.

It is further preferred that the Form II olanzapine polymorph will be administered as the substantially pure Form II olanzapine polymorph.

As used herein "substantially pure" refers to Form II associated with less than about 5% Form I, preferably less than about 2% Form I, and more preferably less than about 1% Form I. Further, "substantially pure" Form II will contain less than about 0.5% related substances, wherein "related substances" refers to undesired chemical impurities or residual solvent or water. In particular, "substantially pure" Form II should contain less than about 0.05% content of acetonitrile, more preferably, less than about 0.005% content of acetonitrile. Additionally, the polymorph of the invention should contain less than 0.5% of associated water.

The polymorph obtainable by the process taught in the '382 patent will be designated as Form I and has a typical x-ray powder diffraction pattern substantially as follows, obtained using a Siemens D5000 x-ray powder diffractometer, wherein d represents the interplanar spacing:

| **d** |
|---|
| 9.9463 |
| 8.5579 |
| 8.2445 |
| 6.8862 |
| 6.3787 |
| 6.2439 |
| 5.5895 |
| 5.3055 |
| 4.9815 |
| 4.8333 |
| 4.7255 |
| 4.6286 |
| 4.533 |
| 4.4624 |
| 4.2915 |
| 4.2346 |
| 4.0855 |
| 3.8254 |
| 3.7489 |
| 3.6983 |
| 3.5817 |
| 3.5064 |
| 3.3392 |
| 3.2806 |
| 3.2138 |
| 3.1118 |
| 3.0507 |
| 2.948 |
| 2.8172 |
| 2.7589 |
| 2.6597 |
| 2.6336 |
| 2.5956 |

A typical example of an x-ray diffraction pattern for Form I is as follows wherein d represents the interplanar spacing and I/I₁ represents the typical relative intensities:

| **d** | **I/I** _{**1**} |
|---|---|
| 9.9463 | 100.00 |
| 8.5579 | 15.18 |
| 8.2445 | 1.96 |
| 6.8862 | 14.73 |
| 6.3787 | 4.25 |
| 6.2439 | 5.21 |
| 5.5895 | 1.10 |
| 5.3055 | 0.95 |
| 4.9815 | 6.14 |
| 4.8333 | 68.37 |
| 4.7255 | 21.88 |
| 4.6286 | 3.82 |
| 4.533 | 17.83 |
| 4.4624 | 5.02 |
| 4.2915 | 9.19 |
| 4.2346 | 18.88 |
| 4.0855 | 17.29 |
| 3.8254 | 6.49 |
| 3.7489 | 10.64 |
| 3.6983 | 14.65 |
| 3.5817 | 3.04 |
| 3.5064 | 9.23 |
| 3.3392 | 4.67 |
| 3.2806 | 1.96 |
| 3.2138 | 2.52 |
| 3.1118 | 4.81 |
| 3.0507 | 1.96 |
| 2.948 | 2.40 |
| 2.8172 | 2.89 |
| 2.7589 | 2.27 |
| 2.6597 | 1.86 |
| 2.6336 | 1.10 |
| 2.5956 | 1.73 |

The x-ray powder diffraction patterns herein were obtained with a copper Kₐ of wavelength 1 = 1.541Å. The interplanar spacings in the column marked "d" are in Angstroms. The typical relative intensities are in the column marked "I/I₁".

As used herein, the term "mammal" shall refer to the Mammalia class of higher vertebrates. The term "mammal" includes, but is not limited to, a human. The term "treating" as used herein includes prophylaxis of the named condition or amelioration or elimination of the condition once it has been established.

Olanzapine is effective over a wide dosage range; however, it is desirable to administer a dosage that is as low as possible. For example, dosages per day of the olanzapine will normally fall within the range of about 1 mg to about 30 mg per day. However, it will be understood that the amount of the compound actually administered will be determined by a physician, in the light of the relevant circumstances including the type of neuro-protective effect or condition to be treated, the age, weight, and response of the individual patient, the severity of the patient's symptoms, and the chosen route of administration, and therefore the above dosage ranges are not intended to limit the scope of the invention in any way. While the present compounds are preferably administered orally to humans susceptible to or suffering from neuro-degeneration, the compounds may also be administered by a variety of other routes such as the transdermal, parenterally, subcutaneous, intranasal, intramuscular and intravenous routes. Such formulations may be designed to provide delayed or controlled release using formulation techniques which are known in the art.

"Ischemia" represents a phenomenon in which tissue is deprived of either partial or total blood flow in conjunction with hypoxia. Reperfusion of such tissue causes additional tissue injury associated with ischemic events to vital organs such as the lung, liver, kidney, heart and small bowel.

As used herein the term "treating" includes prophylaxis of neuro-degeneration in a patient having a tendency to develop such neuro-degeneration, and the amelioration or elimination of the developed neuro-degeneration once it has been established or alleviation of the characteristic symptoms of such neuro-degeneration.

The results of pharmacological studies show that olanzapine has muscarinic cholinergic receptor activity. The compound is active at the dopamine D-1 and D-2 receptors as indicated by an IC50 of less than 1 uM in the 3H-SCH233390 (Billard, et al. Life Sciences **35**:1885 (1984)) and the 3H spiperone (Seeman et al Nature **216**:717 (1976)) binding assays respectively. Further, olanzapine is active at the 5-HT-2 receptor and 5-HT1C receptor. The complex pharmacological profile of the compound provides a medicament which can unexpectedly be useful for the treatment of ischemia and to provide neuro-protective benefits.

Compositions suitable for internal administration contain from about one half (0.5) milligrams to about 50 milligrams olanzapine per unit.

Typical compositions include olanzapine or a pharmaceutically acceptable acid addition salt thereof, associated with a pharmaceutically acceptable excipient which may be a carrier, or a diluent or be diluted by a carrier, or enclosed within a carrier which can be in the., form of a capsule, sachet, paper, or other container. In making the compositions, conventional techniques for the preparation of pharmaceutical compositions may be used. For example, the active compound will usually be mixed with a carrier, or diluted by a carrier, or enclosed within a carrier which may be in the form of a ampoule, capsule, sachet, paper, or other container. When the carrier serves as a diluent, it may be solid, semi-solid, or liquid material which acts as a vehicle, excipient, or medium for the active compound. The active compound can be adsorbed on a granular solid container for example in a sachet. Some examples of suitable carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose and polyvinylpyrrolidone. The formulations may also include wetting agents, emulsifying and suspending agents, preserving agents, sweetening agents, or flavoring agents. The formulations of the invention may be formulated so as to provide quick, sustained, or delayed release of the active ingredient after administration to the patient by employing procedures well known in the art.

The pharmaceutical preparations can be sterilized and mixed, if desired, with auxiliary agents, emulsifiers, salt for influencing osmotic pressure, buffers and/or coloring substances and the like, which do not deleteriously react with the active compounds.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

Generally, olanzapine is dispensed in unit form comprising from about 1 mg to about 30 mg in a pharmaceutically acceptable carrier per unit dosage.

Most preferably, the solid oral formulation is contained in packaging materials which protect the formulation from moisture and light. For example, suitable packaging materials include amber colored high density polyethylene bottles, amber colored glass bottles, and other containers made of a material which inhibits the passage of light. Most preferably, the packaging will include a desiccant pack. The container may be sealed with an aluminum foil blister to provide the desired protection and maintain product stability.

The compositions of this invention may be suitable for administration to an animal. Such animals include both domestic animals, for example livestock, laboratory animals, and household pets, and non-domestic animals such as wildlife. More preferably, the animal is a vertebrate. Most preferably, a compound of this invention shall be administered to a mammal. It is especially preferred that the animal is a domestic mammal or a human. The most preferred mammal is a human. For such purposes, a compound of this invention may be administered as a feed additive.

### Assays

The brain is only about 2% of the total body mass, yet it consumes approximately 20% of all the inspired oxygen. Although neurons depend on oxidative metabolism for survival, a consequence of this process is the production of reactive compounds such as hydrogen peroxide and oxy radicals (Cohen and Werner, 1994). In spite of the high vulnerability of the brain to oxygen radical attack, oxygen free-radical reactions and oxidative damage are in most cases held in check by antioxidant defense mechanisms under basal conditions. Pathological conditions of the central nervous system exist, however, where excessive amounts of oxygen free radicals are produced that impair defense mechanisms. Unchecked, these reactive oxygen species (ROS) can lead to DNA damage, peroxidation of membrane lipids and neuronal death.

Oxidative damage caused by free radical production and lipid peroxidation as well as by products of the arachidonic acid cascade are considered to be primary factors in the acute stage pathology of ischemia. Increases in the amounts of free fatty acids after ischemia and during early reperfusion can provide the substrate for lipid peroxidation and for the formation of products of the arachidonic acid cascade (Clemens, et al., Stroke, Vol. 22, No. 8, Aug. 1991).

Several reviews have been written on the role of oxygen radicals in cerebral ischemia (Braugher and Hall, 1989; Hall and Braugher 1989; Koutos, 1989, Floyd, 1990; Nelson, et al., 1992; Panetta and Clemens, 1993).

In addition, recent reports suggest the involvement of free radicals in the pathogenesis of ALS (Rosen, et al., 1993; McNamara and Fridovich, 1993).

The compounds of the instant invention inhibit the formation of reactive oxygen species in a mammal and are thereby useful for treating conditions and diseases which are believed to be induced by increased free radical production such as global and cerebral ischemia.

Olanzapine can be shown to prevent ischemia-induced neuronal cell damage as demonstrated in the following test system.

### Cerebral Ischemia Model in Rats

Cerebral ischemia is produced in rats by occluding the four arteries that supply blood to the brain according to the following procedure. Male Wistar rats are anesthetized with Metofane and placed into a stereotaxic instrument. A longitudinal incision is made on the dorsal surface of the neck. The neck muscles are reflected to expose the dorsal surface of the spinal column. The two vertebral arteries are exposed where they pass through the first cervical vertebra. Both arteries are permanently occluded by the application of electrocautery. After coagulation of the vertebral arteries, the rat is removed from the stereotaxic instrument and the surgical wound is sutured. Two longitudinal incisions are then made on the ventral surface of the neck. The two common carotid arteries are exposed and dissected free from surrounding nerves and connective tissue. An atraumatic clasp, fabricated mainly from silicone rubber tubing, is placed around each carotid artery in a manner such that the vessel is not traumatized or occluded. An indwelling jugular cannula is implanted into each rat for drug delivery. The surgical wounds are then closed. The atraumatic clasps were designed in such a manner that they could be tightened to occlude the carotid arteries by pulling on a small silastic thread that is allowed to protrude from the wound. Circulation to the brain through the carotids could be restored by relieving the tension on the silastic threads. After the surgery, the rats are allowed to recover for 24 hours.

Cerebral ischemia is induced by tightening the clasps around the carotids. During this time, rats in which ischemia is successfully produced lose the righting reflex and became unresponsive to stimuli. The period of ischemia is 20 minutes, and immediately after the 20 minutes of ischemia, at the time of reperfusion, compounds are administered as an intravenous bolus injection of 10 mg/kg followed by a constant intravenous infusion of 5.0 mg/kg per hour for 20 hours. Five days after the ischemia, the rats are sacrificed, and the brains are perfused, fixed with formalin and processed for histological evaluation.

One of the areas of the brain that is most susceptible to ischemia induced damage both in the rat and the human is the CA₁ pyramidal cell layer of the hippocampus. In animals that remain unresponsive for the 20 minute period of ischemia, the CA₁ pyramidal cell layer is completely destroyed. This layer of cells was examined microscopically in histological sections prepared from the hippocampus. Brain damage was rated according to the following scale:
0 = no damage, completely intact cell layer
1 = mild damage, one-third of CA₁ layer dead
2 = moderate damage, two-thirds of CA₁ layer dead
3 = severe damage, greater than 90% cell death

Damage in 4 sections of the dorsal hippocampus from each brain is assessed in order to obtain an accurate estimate of damage. An average damage score is calculated for each treatment group. Scores from treated groups are compared statistically with scores from control groups which received only the vehicle (phosphate buffered saline) that was used to suspend the compounds. The level of significance is determined using the Mann Whitney-U-test.

Olanzapine is tested in the above-described to illustrate the usefulness for the claimed methods.

The following three tests are useful in predicting the ability of a compound to inhibit free radical formation which is believed to be implicated in disease such as ischemia.

### Lipid Peroxidation Test

Olanzapine is shown to inhibit the formation of lipid peroxides in mammals using the test protocol described by Aruoma, et al., (1990), Free Rad. Res. Comm., 10:143. Olanzapine is tested in the assay cited to illustrate its effectiveness in inhibiting the formation of lipid peroxides.

### Superoxide O₂· Secretion Test

Olanzapine, in addition, is tested for its ability to inhibit superoxide O₂. secretion using the method of Lorico, et al., (1986), Biochem. Pharmacol., 35:2443. Olanzapine is tested in the above mentioned assay to illustrate its usefulness in inhibiting superoxide secretion.

### H₂O₂ Secretion Test

Finally, using the protocol of Root, et al., (1975), J.Clin. Invet., 55:945, herein incorporated by reference, olanzapine is tested for its effectiveness in inhibiting H₂O₂ secretion.

### Pharmaceutical Formulations

Olanzapine is capable of slowing the process of neurodegeneration.

Olanzapine utilized in the present invention is effective over a wide dosage range. Thus, as used herein, the term "therapeutically effective amount" refers to a dosage range of from about 0.5 to about 50 mg/kg of body weight per day. In the treatment of adult humans, the range of about 1.0 to about 25 mg/kg per day, is preferred. However, it will be understood that the amount of the compound actually administered will be determined by a physician, in the light of the relevant circumstances including the choice of compound to be administered, the chosen route of administration, the age, weight, and response of the individual patient, and the severity of the patient's symptoms, and therefore the above dosage ranges are not intended to limit the scope of the invention in any way.

The neurodegenerative diseases and amyotrophic lateral sclerosis are chronic conditions. The term "chronic" means a deteriorating condition of slow progress and long continuance. As such, a chronic neurodegenerating condition is treated when it is diagnosed and continued throughout the course of the disease.

Ischemia represents a phenomenon in which tissue is deprived of either partial or total blood flow in conjunction with hypoxia. It may occur as an acute event or a chronic condition. The term "acute" means an exacerbated condition of short course followed by a period of remission. Thus, the treatment of ischemia induced cell damage contemplates both acute and chronic forms. In an acute event, compound is administered at the onset of symptoms and discontinued when the symptoms disappear. As described above, a chronic condition is treated throughout the course of the disease.

Surprisingly, such experiments demonstrate that olanzapine provides a significant neuro-protective effect.

### Clinical observations.

A double-blind multicenter clinical trial is designed to assess the safety and efficacy of olanzapine. Patients are randomized to olanzapine or placebo. Patients are monitored for evidence of neuro-degeneration using standard methods.

The materials for the present invention can be purchased or prepared by a variety of procedures well known to those of ordinary skill in the art. Olanzapine can be prepared as described by Chakrabarti in U.S. Patent No 5,229,382 ('382), herein incorporated by reference in its entirety. Further, the following preparations illustrate a method for preparing of the especially preferred Form II olanzapine polymorph.

Compound characterization methods include, for example, x-ray powder pattern analysis, thermogravimetric analysis (TGA), differential scanning calorimetery (DSC), titrametric analysis for water, and H¹-NMR analysis for solvent content.

The following examples are provided for purposes of illustration and are not to be construed as limiting the scope of the claimed invention.

### Preparation 1

### Technical Grade olanzapine

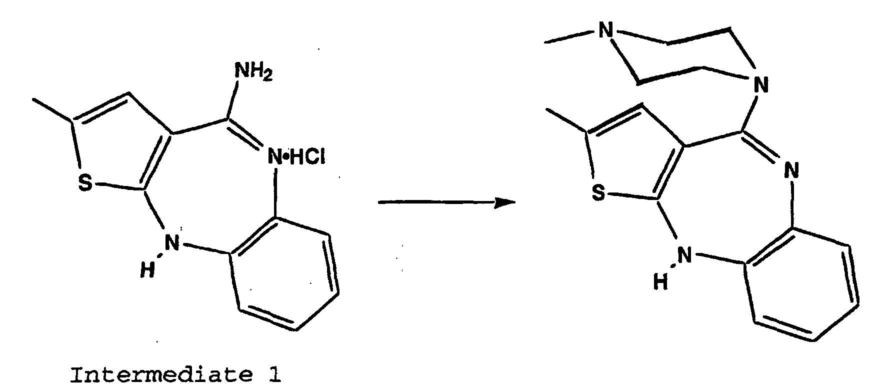

In a suitable three neck flask the following was added:

| | |
|---|---|
| Dimethylsulfoxide (analytical) | 6 volumes |
| Intermediate 1 | 75 g |
| N-Methylpiperazine (reagent) | 6 equivalents |

Intermediate 1 can be prepared using methods known to the skilled artisan. For example, the preparation of the Intermediate 1 is taught in the '382 patent.

A sub-surface nitrogen sparge line was added to remove the ammonia formed during the reaction. The reaction was heated to 120°C and maintained at that temperature throughout the duration of the reaction. The reactions were followed by HPLC until ≤ 5% of the intermediate 1 was left unreacted. After the reaction was complete, the mixture was allowed to cool slowly to 20°C (about 2 hours). The reaction mixture was then transferred to an appropriate three neck round bottom flask and water bath. To this solution with agitation was added 10 volumes reagent grade methanol and the reaction was stirred at 20°C for 30 minutes. Three volumes of water was added slowly over about 30 minutes. The reaction slurry was cooled to zero to 5°C and stirred for 30 minutes. The product was filtered and the wet cake was washed with chilled methanol. The wet cake was dried in vacuo at 45°C overnight. The product was identified as technical olanzapine.
Yield: 76.7%; Potency: 98.1%

### Preparation 2

### Form II olanzapine polymorph

A 270 g sample of technical grade 2-methyl-4-(4-methyl-1-piperazinyl)-10H-thieno[2,3-b][1,5]benzodiazepine was suspended in anhydrous ethyl acetate (2.7 L) . The mixture was heated to 76°C and maintained at 76°C for 30 minutes. The mixture was allowed to cool to 25°C. The resulting product was isolated using vacuum filtration. The product was identified as Form II using x-ray powder analysis.
Yield: 197 g.

The process described above for preparing Form II provides a pharmaceutically elegant product having potency ≥ 97%, total related substances < 0.5% and an isolated yield of > 73%.

### EXAMPLE 1

A portion of the hydroxypropyl cellulose was dissolved in purified water to form a solution for granulation. The remaining hydroxypropyl cellulose (total of 4.0% w/w final tablet weight), which was an extra fine grade, was combined with the olanzapine (1.18% w/w), lactose (79.32% w/w) and a portion of the crospovidone (5% w/w) in a high shear granulator. All ingredients were security sieved prior to addition and dry blended in the granulator. This mixture was then granulated with the hydroxypropyl cellulose solution in the high shear granulator. The granulation was wet sized using standard methods. The wet granulation was then dried in a fluidized bed dryer and sized. The material was then added to a tumble bin mixer.

The running powders consisting of microcrystalline cellulose (granular) (10% w/w), magnesium stearate (0.5% w/w), and the remainder of the crospovidone were added to the sized granulation. The mixture was blended and compressed with the appropriate tooling on tablet compression equipment.

### Subcoating

Hydroxypropyl methylcellulose (10% w/w) was mixed with purified water to form a solution. Core tablets were divided into approximately equal sections and spray coated with the hydroxypropyl methylcellulose solution. The operation was performed in a perforated coating pan.

### Coating of Core Tablets

Color Mixture White (hydroxypropyl methylcellulose, polyethylene glycol, polysorbate 80, and titanium dioxide) was mixed with purified water to form the coating suspension. Subcoated tablets were divided into approximately equal sections and spray coated with the coating suspension described above. The operation was performed in a perforated coating pan.

The coated tablets were lightly dusted with carnauba wax and imprinted with appropriate identification.

## Claims

1. Use of olanzapine or a pharmaceutically acceptable salt or solvate thereof for the manufacture of a medicament for treating neurodegeneration in a mammal in need of such treatment.

2. Use of Claim 1 wherein olanzapine is Form II olanzapine polymorph having a typical x-ray diffraction pattern as follows, wherein d represents the interplanar spacing:
| **d** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

3. Use of Claim 2 wherein the dosage of olanzapine is from about 5 mg to about 30 mg per day.

## Patentansprüche

1. Verwendung von Olanzapin oder eines pharmazeutisch annehmbaren Salzes oder Solvats hiervon zur Herstellung eines Arzneimittels zur Behandlung von Neurodegeneration bei einem Säuger, der einer solchen Behandlung bedarf.

2. Verwendung nach Anspruch 1, worin das Olanzapin ein Olanzapinpolymorph der Form II ist, welche das folgende typische Röntgenbeugungsmuster aufweist, worin d die Interplanarabstände bedeutet:
| d | d (Fortsetzung) |
|---|---|
| 10,2689 | 3,9873 |
| 8,577 | 3,7206 |
| 7,4721 | 3,5645 |
| 7,125 | 3,5366 |
| 6,1459 | 3,3828 |
| 6,071 | 3,2516 |
| 5,4849 | 3,134 |
| 5,2181 | 3,0848 |
| 5,1251 | 3,0638 |
| 4,9874 | 3,0111 |
| 4,7665 | 2,8739 |
| 4,7158 | 2,8102 |
| 4,4787 | 2,7217 |
| 4,3307 | 2,6432 |
| 4,2294 | 2,6007 |
| 4,141 | |

3. Verwendung nach Anspruch 2, worin die Dosis an Olanzapin etwa 5 mg bis etwa 30 mg pro Tag beträgt.

## Revendications

1. Utilisation de l'olanzapine ou d'un sel ou solvate pharmaceutiquement acceptable de celle-ci pour la fabrication d'un médicament pour le traitement de la neurodégénérescence chez un mammifère nécessitant un tel traitement.

2. Utilisation selon la revendication 1 dans laquelle l'olanzapine est l'olanzapine polymorphe sous Forme II ayant un digramme de diffraction aux rayons X typique comme suit, dans lequel d représente la distance interréticulaire:
| d |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

3. Utilisation selon la revendication 2, dans laquelle la posologie de l'olanzapine est d'environ 5 mg à environ 30 mg par jour.
